# EUROPÄISCHE PATENTSCHRIFT

(11) **EP 2 914 160 B1**
(45) Veröffentlichungstag und Bekanntmachung des Hinweises auf die Patenterteilung: **10.07.2019**
(21) Anmeldenummer: 13785435.2
(22) Anmeldetag: 29.10.2013
(51) Int. Cl.: A61B 5/00, A61N 1/375, A61B 5/0478, A61B 5/0492

(54) **MIKROELEKTRODENARRAY FÜR EIN ELEKTROKORTIKOGRAMM (ECOG)**
MICROELECTRODE ARRAY FOR AN ELECTROCORTICOGRAM (ECOG)
RÉSEAU DE MICRO-ÉLECTRODES POUR UN ELECTROCORTICOGRAMME (ECOG)

(30) Priorität: 30.10.2012 DE 102012110358
(43) Veröffentlichungstag der Anmeldung: 09.09.2015
(60) Teilanmeldung: 16177974.9
(73) Patentinhaber: Leibniz-Institut für Neurobiologie Magdeburg, 39118 Magdeburg (DE); Otto-von-Guericke-Universität Magdeburg, 39106 Magdeburg (DE)
(72) Erfinder: OHL, Frank, 39171 Osterweddingen (DE); LIPPERT, Michael, 39104 Magdeburg (DE); SCHMIDT, Bertram, 78052 Villingen-Schwenningen (DE); DECKERT, Martin, 39104 Magdeburg (DE); HIRSCH, Sören, 14789 Wusterwitz (DE)
(74) Vertreter: Günther, Constantin
(86) Internationale Anmeldenummer: PCT/EP2013/072638
(87) Internationale Veröffentlichungsnummer: WO 2014/067963

(56) Entgegenhaltungen:
- DE-A1-102012 002 663
- US-A- 5 024 226
- US-A1- 2005 075 680
- US-A1- 2011 237 921
- US-A1- 2012 253 261
- US-B2- 7 729 773

## Beschreibung

Die Erfindung betrifft ein Mikroelektrodenarray mit einer Vielzahl von Elektroden zur elektrischen Messung von Hirnströmen gemäß dem Anspruch 1.

Aus der US 7,729,773 B2 ist ein neurales Stimulations- und optisches Überwachungssystem sowie entsprechende Verfahren dazu bekannt.

Es gibt bereits Ansätze zur Miniaturisierung von Elektroden zur Messung von Hirnströmen insbesondere in Form von sogenannten Mikroelektrodenarrays. Als Array bezeichnet man dabei eine Anordnung einer Vielzahl von Elektroden, die regelmäßig oder unregelmäßig angeordnet sein können, z.B. in einer Matrixanordnung. Mit solchen Elektroden bzw. solchen Mikroelektrodenarrays können elektrische Signa-le des Gehirns eines Lebewesens aufgenommen werden, wobei sowohl auf der Hirnoberfläche als auch innerhalb des Hirns Messungen möglich sind. Insbesondere kann mit solchen Mikroelektrodenarrays ein Elektrokortikogramm (ECoG) auf-genommen werden.

Es gibt außerdem Entwicklungen im Bereich der sogenannten "Optogenetik", durch Licht Neurone im Hirn zu stimulieren, sofern diese bestimmte lichtsensitive Kanalproteine exprimieren. Typischerweise wird das für die Stimulation erforderliche Licht von außen durch Glasfasern oder an den Schädel montierte Leuchtdioden (LEDs) ins Hirn eingestrahlt.

Es ergeben sich hierbei mehrere Probleme, nämlich dass die Implantation der Lichtquelle und des Mikroelektrodenarrays räumlich viel Platz beansprucht, die Versuchsobjekte mit einer Glasfaserleitung an externe Technik angebunden sind und die genaue räumliche Ausrichtung von Lichtquelle und den einzelnen Elektroden zueinander schwierig ist. Der gesamte Implantationsvorgang ist durch die Notwendigkeit mehrerer Schritte und Technologien relativ kompliziert. Der Erfindung liegt die Aufgabe zugrunde, dies zu vereinfachen.

Die Aufgabe wird gelöst durch ein Mikroelektrodenarray gemäß Anspruch 1. Ein Vorteil der Erfindung ist es, dass die elektrische Lichtquelle bzw. die elektrischen Lichtquellen in das Mikroelektrodenarray integriert sind und somit nicht als separate Teile positioniert und implantiert werden müssen. Auch die elektrische Kontaktierung wird vereinfacht, da gemeinsame Leitungen, z.B. in Form eines Flachbandkabels, verwendet werden können. Ein weiterer Vorteil der Erfindung besteht darin, dass die elektrische Lichtquelle bzw. die elektrischen Lichtquellen in Bezug zu den Elektroden räumlich fixiert und exakt positioniert sind, wobei die exakte Positionierung bereits durch die Herstellung des Mikroelektrodenarrays vorgegeben ist. Daher ist das erfindungsgemäße Mikroelektrodenarray wesentlich anwendungsfreundlicher als bekannte Einrichtungen. Durch die Erfindung wird es möglich, kortikale Neuronen mit Hilfe von einer oder mehreren integrierten elektrischen Lichtquellen räumlich präzise und strukturiert optisch zu stimulieren und gleichzeitig die dadurch ausgelösten und spontanen Hirnströme elektrophysiologisch durch die mikrostrukturierten Elektroden abzuleiten.

Ein weiterer Vorteil besteht darin, dass die Möglichkeit besteht, Hirnbereiche mit verschiedenen räumlichen Mustern zu stimulieren, beispielsweise um topographische Beziehungen im Hirn auszumessen und zu stimulieren, z.B. für die Tonotopie, Somatotopie, Retinotopie.

Gemäß einer vorteilhaften Weiterbildung der Erfindung weist das Mikroelektrodenarray ein folienartiges, dünnes Substrat auf. Das Substrat kann insbesondere ein flexibles Substrat sein. Geeignete Materialien zur Herstellung des Substrats sind z.B. Folien aus Polyimid, Parylen, Polydimethylsiloxan (PDMS) oder Polyurethan. Es ist insbesondere vorteilhaft, ein optisch hinreichend transparentes Material, z.B. ein Folienmaterial, für das Substrat zu verwenden, so dass das von den elektrischen Lichtquellen abgegebene Licht das Substrat durchdringen kann. Das Substrat kann insbesondere mehrschichtig aus denselben oder verschiedenen Materialien aufgebaut sein, z.B. nach Art einer Sandwichstruktur.

Gemäß einer vorteilhaften Weiterbildung der Erfindung sind die Elektroden über die flächige Ausdehnung des Substrats verteilt an der Oberfläche des Substrats oder in dem Substrat angeordnet. Die Elektroden können z.B. als elektrisch leitende Beschichtung, z.B. als Metall-Beschichtung, auf der Oberfläche des Substrats oder als Metallschicht im Substrat ausgebildet sein. Gemäß einer vorteilhaften Weiterbildung der Erfindung sind eine Vielzahl von elektrischen Lichtquellen der Stimulationseinheit über die flächige Ausdehnung des Substrats verteilt an der Oberfläche des Substrats oder in dem Substrat angeordnet.

Die elektrischen Lichtquellen können insbesondere als Leuchtdioden ausgebildet sein, wobei anorganische Leuchtdioden oder organische Leuchtdioden (OLEDs) Anwendung finden können, auch in Kombination miteinander. Des Weiteren kann die elektrische Lichtquelle bzw. können die elektrischen Lichtquellen als phosphoreszierende organische Leuchtdioden (PHOLEDs) in den Aufbau des Mikroelektrodenarrays integriert sein oder in Kombination mit den zuvor genannten Lichtquellen Verwendung finden. Der verbesserte Wirkungsgrad der PHOLEDs im Vergleich zu anderen Lichtquellen ist vorteilhaft, da bei gleicher Lichtintensität eine geringere Wärmeentwicklung induziert und das Risiko einer Gewebeschädigung bzw. Schädigung von Hirnbereichen durch Erwärmung reduziert wird.

Gemäß der Erfindung ist vorgesehen, dass die elektrische Lichtquelle bzw. die elektrischen Lichtquellen nach einem festen vorgegebenen Schema relativ zu den Elektroden angeordnet und über die flächige Ausdehnung des Substrats verteilt sind. Hierdurch ist eine fest vorgegebene Zuordnung zwischen den Elektroden und den elektrischen Lichtquellen vorgegeben, so dass die über die Elektroden aufgenommene Stimulation der Neuronen mittels der elektrischen Lichtquellen in einzelnen Stimulationssignalen eindeutig zugeordnet werden kann, so dass eine eindeutige Korrelation bestimmt werden kann.

Gemäß einer vorteilhaften Weiterbildung der Erfindung sind auf der Oberfläche des Substrats oder in dem Substrat elektrisch leitende Strukturen gebildet, die die Elektroden, elektrische Verbindungsleitungen zu den Elektroden und/oder elektrische Verbindungsleitungen zu der elektrischen Lichtquelle bzw. den elektrischen Lichtquellen bilden. Dies hat den Vorteil, dass die elektrisch leitenden Strukturen, z.B. in Form von einer oder mehreren Metallschichten, in dem Substrat direkt eingebettet sein können oder darauf aufgebracht sein können, z.B. aufgedampft sein können.

Die elektrisch leitenden Strukturen können z.B. in Form einer oder mehrerer Metallisierungsebenen im Substrat bereit gestellt werden. Die Metallisierungsebene bzw. die Metallisierungsebenen, die Elektroden und/oder Verbindungsleitungen bildet, kann direkt zur Stromversorgung der Lichtquellen benutzt werden. Es kann auch eine zusätzliche gegebenenfalls dickere und/oder strukturierte Metallschicht zur Stromversorgung in dem Mikroelektrodenarray vorgesehen werden.

Gemäß der Erfindung weist das Mikroelektrodenarray eine Sensorseite auf, die dazu eingerichtet ist, in Kontakt mit der Hirnoberfläche eines zu untersuchenden Lebewesens gebracht zu werden. Dabei sind eine, mehrere oder alle elektrischen Lichtquellen in größerem Abstand von der Sensorseite angeordnet als die Elektroden. Dies ermöglicht eine günstige Unterbringung der Lichtquellen. Insbesondere ist es möglich, eine, mehrere oder alle elektrischen Lichtquellen auf der der Sensorseite abgewandten Oberfläche des Substrats anzuordnen. Des Weiteren kann durch die vorteilhafte Anordnung der elektrischen Lichtquelle bzw. der elektrischen Lichtquellen in relativem Abstand zur Sensorseite der Wärmeeintrag in die stimulierten Hirnbereiche verringert werden, um eine Gewebeschädigung zu unterbinden

Die elektrischen Lichtquellen können insbesondere als SMD-Bauelemente, als Dies oder als Dünnschichtelemente auf dem Substrat oder innerhalb des Substrats angeordnet sein.

Gemäß einer vorteilhaften Weiterbildung der Erfindung ist zumindest die Sensorseite des Mikroelektrodenarrays elektrisch und biologisch passiviert. Hierdurch werden unerwünschte gegenseitige Effekte mit dem Hirngewebe vermieden. Die Passivierung kann z.B. als Isolationsschicht ausgebildet sein.

Gemäß der Erfindung sind die Elektroden in das Material des Substrats integriert, z.B. darin eingebettet. Das Substrat weist dann an einer Sensorseite, die dazu eingerichtet ist, in Kontakt mit der Hirnoberfläche eines zu untersuchenden Lebewesens gebracht zu werden, zu den Elektroden führende Öffnungen auf. Hierdurch kann der elektrische Kontakt zu den Elektroden auch bei in das Material des Substrats integrierten Elektroden hergestellt werden. Die Integration der Elektroden in das Substrat hat den Vorteil, dass diese platzsparend darin angeordnet sind und besser vor Schädigungen geschützt sind.

Gemäß einer vorteilhaften Weiterbildung der Erfindung sind eine, mehrere oder alle Elektroden als ECoG-Elektroden ausgebildet.

Gemäß einer vorteilhaften Weiterbildung der Erfindung weist das Mikroelektrodenarray eine oder mehrere Stimulationselektroden zur Stimulation von Hirnbereichen mit elektrischen Signalen auf. Dies hat den Vorteil, dass zusätzlich eine Stimulation über elektrische Signale, die direkt galvanisch auf Hirnbereiche übertragen werden, möglich ist. Das Mikroelektrodenarray kann damit auch kombinierte Stimulationen mit optischen und elektrischen Signalen durchführen. Hierdurch werden die Anwendungsmöglichkeiten des Mikroelektrodenarrays erweitert.

Gemäß einer vorteilhaften Weiterbildung der Erfindung sind eine oder mehrere Elektroden zur elektrischen Messung von Hirnströmen zugleich Stimulationselektroden zur Stimulation von Hirnbereichen mit elektrischen Signalen. In diesem Fall besteht ein Vorteil darin, dass das Mikroelektrodenarray mit einer Vielzahl von Elektroden ausgebildet werden kann und diese wahlweise als Mess-Elektroden oder als Stimulationselektroden verwendet werden. Gemäß einer weiteren vorteilhaften Weiterbildung der Erfindung werden Elektroden zeitweise zur elektrischen Messung von Hirnströmen und zeitweise als Stimulationselektroden zur Stimulation von Hirnbereichen mit elektrischen Signalen eingesetzt. Eine entsprechende Steuerung der Funktion der jeweiligen Elektrode kann über eine Steuerelektronik erfolgen.

Gemäß einer vorteilhaften Weiterbildung der Erfindung sind in relativer Nähe zu einer, mehreren oder allen elektrischen Lichtquellen jeweils ein oder mehrere weitere elektrische und/oder elektronische Bauteile, insbesondere Sensorbauteile, angeordnet. Dies hat den Vorteil, dass über das oder die weiteren elektrischen bzw. elektronischen Bauteile je nach deren Ausführung weitere Möglichkeiten der Beeinflussung des Gewebes bzw. von Hirnbereichen möglich sind oder, wenn es sich um Sensorbauteile handelt, weitere Daten erfasst werden können. So kann ein weiteres elektrisches bzw. elektronisches Bauteil oder eine Gruppe solcher Bauteile jeweils einer elektrischen Lichtquelle zugeordnet sein und in deren Nähe angeordnet sein, z. B. neben der Lichtquelle oder um die Lichtquelle herum verteilt.

Gemäß einer vorteilhaften Weiterbildung der Erfindung sind in relativer Nähe zu einer, mehreren oder allen elektrischen Lichtquellen jeweils ein oder mehrere weitere elektrische und/oder elektronische Sensorbauteile, die jeweils ein elektrisches Sensorsignal abgeben, derart angeordnet, dass wenigstens eine von der elektrischen Lichtquelle oder den elektrischen Lichtquellen beeinflusste physikalische Größen durch das jeweilige Sensorbauteil erfassbar sind. Dies hat den Vorteil, dass durch solche Sensorbauteile von den Lichtquellen beeinflusste physikalische Größen überwacht werden können, wie z. B. das von der jeweiligen Lichtquelle abgegebene Licht, das direkt vor Ort ggf. unter Berücksichtigung von Reflektions- und/oder Absorptionseigenschaften des Hirngewebes erfasst werden kann. Auch andere von den elektrischen Lichtquellen beeinflusste physikalische Größen, wie z. B. die örtliche Temperatur, können erfasst werden. Die durch die Sensorbauteile erfassten Daten können z. B. auf einem Anzeigegerät (Display) angezeigt werden und damit visuell überwacht werden. Es kann auch eine automatische Überwachung auf Einhaltung zulässiger Grenzwerte durchgeführt werden, z. B. derart, dass bei Über- oder Unterschreiten bestimmter Grenzwerte automatisch ein Hinweissignal an den Benutzer erzeugt wird. Vorteilhaft ist es außerdem möglich, die erfassten Sensorsignale wiederum zur Beeinflussung der Lichtquellen bzw. des damit abgegebenen Lichts einzusetzen, so dass eine Steuerung der Lichtquellen in Abhängigkeit von den Sensorsignalen durchgeführt werden kann. Es kann auch ein vollständiger Regelkreis zur Regelung der elektrischen Lichtquellen in Abhängigkeit von Sensorsignalen der elektrischen und/oder elektronischen Sensorbauteile realisiert werden. Die Lichtquellen können dabei z. B. durch Variation elektrischer Parameter wie Strom und/oder Spannung gesteuert bzw. geregelt werden. Die Beeinflussung der elektrischen Parameter kann auch durch gepulste Signale erfolgen, deren Tastverhältnis variiert wird.

Gemäß einer vorteilhaften Weiterbildung der Erfindung weisen eine, mehrere oder alle elektrischen Lichtquellen der jeweiligen Lichtquelle zugeordnete ein oder mehrere Sensorbauteile auf, die jeweils ein elektrisches Sensorsignal abgeben, das der beeinflussten physikalischen Größe einer bestimmten Lichtquelle zuordenbar ist. Durch eine solche Zuordnungsmöglichkeit der von der Lichtquelle beeinflussten physikalischen Größe zu der Lichtquelle kann eine unabhängige, differenzierte Steuerung und/oder Regelung der jeweiligen einzelnen Lichtquelle gezielt durchgeführt werden, was insbesondere bei einer großen Anzahl elektrischer Lichtquellen vorteilhaft ist.

Gemäß einer vorteilhaften Weiterbildung der Erfindung sind einer, mehrere oder alle der Sensorbauteile als Temperatursensoren ausgebildet. Dies hat den Vorteil, dass die lokale Temperatur im Gewebe bzw. in Hirnbereichen gemessen werden kann. Insbesondere kann eine Messung der durch die Lichtquelle bzw. die Lichtquellen bedingten Temperaturerhöhung im Gewebe bzw. in den Hirnbereichen durchgeführt werden. In Verbindung mit der zuvor erwähnten Steuerung und/oder Regelung der Lichtquelle kann sichergestellt werden, dass die von den Lichtquellen verursachte Temperaturerhöhung in solchen Grenzen gehalten wird, dass eine Schädigung des Gewebes vermieden wird. Die Temperaturerhöhung kann somit regelungstechnisch auf zulässige Grenzwerte beschränkt werden.

Mit solchen Temperatursensoren kann auch eine Erwärmung von Stimulationselektroden, z.B. verursacht durch die Lichtquellen, erfasst werden.

Derartige Temperatursensoren können beispielsweise als Mäander ausgeführt sein und als Messprinzip die Temperaturabhängigkeit des elektrischen Widerstands der Metallisierung nutzen. Die Temperatursensoren können z. B. aus Platin hergestellt sein, wobei auch die Elektroden des Mikroelektrodenarrays aus Platin hergestellt sein können. Auch andere Ausführungsformen von Temperatursensoren sind einsetzbar.

Der Vorteil der Integration von Temperatursensoren basiert auf der zur Stimulation von Hirnbereichen mit optischen Signalen simultanen Temperaturmessungen in relativer Nähe zu der elektrischen Lichtquelle bzw. den elektrischen Lichtquellen, um das Risiko einer Gewebeschädigung bzw. Schädigung von Hirnbereichen durch unzulässige Erwärmung zu kontrollieren und/oder zu unterbinden.

Gemäß einer vorteilhaften Weiterbildung der Erfindung sind einer, mehrere oder alle der Sensorbauteile als lichtempfindliche Sensoren ausgebildet. Es können hierfür im Prinzip alle bekannten Arten lichtempfindlicher Sensoren eingesetzt werden, wie z. B. lichtempfindliche Widerstände oder Fotodioden. Besonders vorteilhaft sind anorganische und/oder organische Fotodioden, da sie gut in das Mikroelektrodenarray integrierbar sind. Der Einsatz solcher lichtempfindlichen Sensoren als weitere Sensorbauteile des Mikroelektrodenarrays hat den Vorteil, dass eine simultane Messung der Lichtintensität der stimulierenden optischen Signale möglich wird, welche die Korrelation der zur Stimulation kortikaler Neuronen bzw. von den Hirnbereichen aufgewendeten Lichtintensität zu der über die Elektroden aufgenommenen neuronalen Aktivität ermöglicht. Durch entsprechende computergestützte Auswertung der Signale der Elektroden sowie der Signale der lichtempfindlichen Sensoren können korrigierte Elektrodensignale bestimmt werden, die um unerwünschte Nebeneffekte wie z. B. Abschattungen von Lichtquellen oder Reflektionen des abgegebenen Lichts bereinigt sind. Gleichzeitig können die Absorption sowie die Streuung der stimulierenden optischen Signale durch kortikales Gewebe bestimmt werden. In Verbindung mit der zuvor erwähnten Steuerung bzw. Regelung der elektrischen Lichtquellen in Abhängigkeit von den Sensorsignalen kann auch eine automatische Korrekturfunktion der Lichtabgabe der Lichtquellen an gewünschte Lichtabgabewerte durchgeführt werden, indem die Sensorsignale daraufhin geprüft werden, ob die gewünschten Lichtabgabewerte der Lichtquellen erreicht werden, und falls dies nicht der Fall ist, korrigierend durch Steuerung bzw. Regelung der elektrischen Lichtquellen eingegriffen werden.

Die genannten Steuerung- bzw. Regelungsfunktionen können vorteilhaft durch eine elektronische Steuereinheit realisiert werden, die die Steuer- bzw. Regelfunktionen hardwaremäßig und/oder durch Softwaresteuerung durchführt, z. B. mit einem Mikroprozessor oder Mikrocontroller.

Gemäß der Erfindung sind die weiteren elektrischen und/oder elektronischen Bauteile nach einem festen, vorgegebenen Schema relativ zu den Lichtquellen angeordnet und über die flächige Ausdehnung des Substrats verteilt an der Oberfläche des Substrats oder in dem Substrat angeordnet. Des ermöglicht z. B. eine flächendeckende sensorische Erfassung von Größen über das gesamte Mikroelektrodenarray.

Gemäß einer vorteilhaften Weiterbildung der Erfindung sind auf der Oberfläche des Substrats oder in dem Substrat elektrisch leitende Strukturen gebildet, die elektrische Verbindungsleitungen zu dem weiteren elektrischen und/oder elektronischen Bauteil oder zu den weiteren elektrischen und/oder elektronischen Bauteilen bilden. Auf diese Weise können z. B. die Temperatursensoren und/oder die lichtempfindlichen Sensoren ohne externe Leitungen elektrisch kontaktiert werden. Die elektrischen Verbindungsleitungen können z. B. in gleicher Weise realisiert werden wie bei den elektrischen Lichtquellen.

Eine direkte Einbettung von elektrischen Verbindungsleitungen in dem Substrat kann z. B. durch Abscheidung metallischer Werkstoffe, z. B. durch Kathodenzerstäubung, durchgeführt werden. Eine solche Einbettung der elektrischen Verbindungsleitungen auf dem Substrat erhöht den Integrationsgrad und erlaubt damit eine erweiterte Funktionalität des Mikroelektrodenarrays, z. B. zur Temperaturmessung und Messung der Lichtintensität, ohne bauliche Vergrößerung.

Die elektrisch leitenden Strukturen, die z. B. als Metallisierungsebenen ausgebildet sind, können somit z. B. direkt zur Stromversorgung des Temperatursensors bzw. der Temperatursensoren und/oder des lichtempfindlichen Sensors bzw. der lichtempfindlichen Sensoren genutzt werden. Es ist dabei vorteilhaft, die Anzahl funktionsintegrierender Metallisierungsebenen nicht zu groß zu wählen, z. B. nur eine Metallisierungsebene zu verwenden. Hierdurch kann die erreichbare mechanische Flexibilität bei geringer Steifigkeit des Mikroelektrodenarrays verbessert werden, wodurch zugleich eine verbesserte Anpassungsfähigkeit des Mikroelektrodenarrays an die Topografie der Hirnoberfläche ermöglicht wird.

Gemäß einer vorteilhaften Weiterbildung der Erfindung sind eines, mehrere oder alle weiteren elektrischen und/oder elektronischen Bauteile als SMD-Bauteile, als Dies oder als Dünnschichtelemente auf dem Substrat oder innerhalb des Substrats angeordnet. Dies hat den Vorteil, dass die elektrischen bzw. elektronischen Bauteile platzsparend an oder in dem Substrat angeordnet sind und besser vor Schädigungen geschützt werden können. Die Annäherung eines, mehrerer oder aller elektrischen und/oder elektronischen Bauteile, insbesondere der Sensorbauteile wie der Temperatursensoren, im Substrat an die Sensorseite erlaubt die Erfassung von physikalischen Größen, insbesondere eine Temperaturmessung, die einen hohen Korrelationsgrad mit tatsächlichen physikalischen Größen stimulierter Hirnbereich aufweist.

Gemäß einer vorteilhaften Weiterbildung der Erfindung weist das Mikroelektrodenarray eine Empfangsvorrichtung zum drahtlosen Empfang von auf das Mikroelektrodenarray abgestrahlten elektromagnetischen Wellen auf. Das Mikroelektrodenarray ist mit der für seinen Betrieb notwendigen elektrischen Energie über die Empfangsvorrichtung drahtlos versorgbar und/oder ein Akkumulator des Mikroelektrodenarrays ist über die Empfangsvorrichtung drahtlos aufladbar. Die Empfangsvorrichtung kann z. B. als auf dem Substrat oder innerhalb des Substrats vorgesehene Spule ausgebildet sein, die z. B. aus dem Material der elektrisch leitenden Strukturen gebildet ist, aus denen die bereits erwähnten elektrischen Verbindungsleitungen zu den Lichtquellen oder den sonstigen elektrischen und/oder elektronischen Bauteilen gebildet sein können. Dies hat den Vorteil, dass keine Kabelverbindung zur Versorgung des Mikroelektrodenarrays mit elektrischer Energie erforderlich ist. Stattdessen kann z. B. eine in eine flexible Matte eingebettete Energiesendespule von außen auf die Hautoberfläche oder das Haar eines Patienten aufgelegt werden und von dieser über einen Hochfrequenzsignal das Mikroelektrodenarray über die Empfangsvorrichtung drahtlos mit elektrischer Energie versorgt werden. Ein ggf. in dem Mikroelektrodenarray vorhandener Akkumulator kann dadurch auch aufgeladen werden. Es kann z. B. ein Akkumulator vom Lithiumpolymer-Typ in Form einer flachen Schicht in dem Mikroelektrodenarray vorgesehen sein.

Gemäß einer vorteilhaften Weiterbildung der Erfindung weist das Mikroelektrodenarray eine drahtlos arbeitende Signalübertragungseinrichtung auf, die zum drahtlosen Übertragen von Signalen, insbesondere in Form von Daten, von dem Mikroelektrodenarray zu einer Signalempfangseinrichtung und/oder zum drahtlosen Übertragen von Signalen, insbesondere in Form von Daten, von einer Signalsendeeinrichtung zu dem Mikroelektrodenarray eingerichtet ist. Dies hat den Vorteil, dass keine Kabelverbindungen für die Signalübertragung bzw. die Datenübertragung erforderlich sind. Die Signalübertragungseinrichtung kann z. B. nach einem der bekannten Standards ausgebildet sein, z. B. als Bluetooth-Signalübertragungseinrichtung. Die Verwendung solcher kabellosen Verbindungen erlaubt die Senkung des Inflammationsrisikos nach erfolgter Implantation des Mikroelektrodenarrays. Der Vorteil beruht auf dem Wegfall von Kabeldurchführungen, wie z. B. Flachbandkabel, durch den Skalp. Insbesondere kann hierdurch auch das Infektionsrisiko reduziert werden. Die erforderlichen elektronischen Komponenten, z. B. Verstärker, Impedanzwandler, Filter, Multiplexer, Analog/Digital-Wandler, Energiespeicher können z. B. in einer anwendungsspezifischen integrierten Schaltung (ASIC) ganz oder teilweise zusammengeführt sein.

Gemäß einer vorteilhaften Weiterbildung der Erfindung sind eine, mehrere oder alle Elektroden in einer jeweils erhabenen Säulenstruktur integriert, welche die einzelnen Elektroden auf der Sensorseite von der Substratebene beabstandet. Dies hat den Vorteil, dass die Elektroden auf der Sensorseite etwas aus dem Substrat herausstehen können und damit besser bzw. dichter an zu sensierende Positionen an der Hirnoberfläche herangeführt werden können. Ferner kann hierdurch der Abstand zwischen den elektrischen Lichtquellen und der Hirnoberfläche vergrößert werden, so dass der Wärmeeintrag in die Hirnoberfläche reduziert wird. Die mikrotechnologische Fabrikation der Säulenstrukturen kann z. B. lithographische Maskierung und nasschemische Strukturierung einer metallischen Hartmaske erfolgen, wobei die Strukturübertragung in das Substrat beispielsweise mit Hilfe eines Trockenätzprozesses realisiert wird. Die Nutzung einer dickeren Substratfolie und/oder die sukzessive Abscheidung mehrerer Substratdünnschichten erlaubt die Herstellung von Säulenstrukturen mit variierenden Abmessungen. Kapillare Blutgefäße und biologische Strukturen auf der Hirnoberfläche, die die Annäherung planarer Mikroelektrodenarrays an die Hirnoberfläche limitieren, können durch derartige erhabene Säulenstrukturen überwunden werden. Das flexible Substratmaterial erlaubt dabei ein lokales Umgehen biologischer Strukturen. Der Vorteil derartiger Elektrodenstrukturen basiert demzufolge auf der Annäherung der Elektroden an die Hirnoberfläche, ohne diese zu penetrieren, und resultiert in einer verbesserten räumlichen Auflösung von Ableitung und Stimulation. Ein weiterer Vorteil ist, dass die dreidimensionalen Säulenstrukturen das Verrutschen implantierter Mikroelektrodenarrays unterbinden.

Gemäß einer vorteilhaften Weiterbildung der Erfindung sind eine, mehrere oder alle Säulenstrukturen aus dem Substratmaterial gefertigt. Dies erlaubt einen effizienten Herstellungsprozess des Mikroelektrodenarrays. Ferner ist die biologische Verträglichkeit weiterhin gegeben, da keine weiteren Materialien erforderlich sind.

Gemäß einer vorteilhaften Weiterbildung der Erfindung weisen eine, mehrere oder alle Säulenstrukturen eine jeweils umlaufende Dichtlippe auf, die die aktive Elektrodenfläche der in die Säulenstruktur integrierten Elektrode lateral begrenzt. Hierbei kann das Substrat wiederum an der Sensorseite zu den Elektroden führende Öffnungen aufweisen, die die aktiven Elektrodenflächen freigeben. Die umlaufenden Dichtlippen trennen die Elektrodenflächen von der umgebenden Zerebrospinalflüssigkeit, verhindern Ausgleichsströme zwischen den Elektroden und stellen den direkten Kontakt zur Hirnoberfläche her. Dies gewährleistet eine höchstmögliche räumliche Auflösung und beste Signal-Rausch-Abstände derartiger nicht penetrierender Mikroelektrodenarrays.

Gemäß einer vorteilhaften Weiterbildung der Erfindung sind Durchgangsöffnungen im Substrat des Mikroelektrodenarrays angeordnet, die dazu geeignet sind die Diffusion von pharmakologischen Substanzen ins Gewebe zu erlauben und/oder eine oder mehrere penetrierende Tiefenelektroden in das Gewebe einzubringen. Die Durchgangsöffnungen sind vorteilhaft an Positionen angeordnet, an denen nicht gerade die Elektroden, die elektrischen Lichtquellen oder die anderen elektrischen und/oder elektronischen Bauteile des Mikroelektrodenarrays angeordnet sind. So können die Durchgangsöffnungen z. B. in der Nähe der Elektroden, der elektrischen Lichtquelle bzw. den elektrischen Lichtquellen, dem weiteren elektrischen und/oder elektronischen Bauteil bzw. den Bauteilen im Substrat angeordnet sein. Die Durchgangsöffnungen erlauben es z. B. pharmakologische Substanzen z. B. für optogenetische Anwendungen ins Gewebe einzubringen. Es können auch eine oder mehrere penetrierende Tiefenelektroden in das kortikale Gewebe eingebracht werden. Hierdurch werden die Anwendungsmöglichkeiten des Mikroelektrodenarrays grundlegend erweitert. Gleichzeitig bedingen die Durchgangsöffnungen Kapillareffekte, die das Ansaugen des Mikroelektrodenarrays an die Hirnoberfläche und die Verdrängung umgebender Zerebrospinalflüssigkeit vorteilhaft beeinflussen.

Gemäß einer vorteilhaften Weiterbildung der Erfindung sind mehrere oder alle Durchgangsöffnungen nach einem festen, vorgegebenen Muster über die flächige Ausdehnung des Substrats verteilt angeordnet.

Gemäß einer vorteilhaften Weiterbildung der Erfindung sind eine oder mehrere Tiefenelektroden oder wenigstens ein Tiefenelektrodenarray zur simultanen elektrischen Messung von Hirnströmen tieferer Hirnbereiche und/oder der elektrischen sowie optischen Stimulation von tieferen Hirnbereichen in die Durchgangsöffnungen eingeführt. Als Tiefenelektrodenarray wird eine Anordnung von Tiefenelektroden verstanden, die an einem gemeinsamen Träger an festen Positionen zueinander angeordnet sind. Hierdurch werden die Anwendungsmöglichkeiten des Mikroelektrodenarrays grundlegend erweitert. Dies hat den Vorteil, dass der Signalraum auf Grund der Tiefeninformationen vergrößert und entsprechende Korrelationen in Ableitung und Stimulation eruiert werden können.

Die eingangs genannte Aufgabe wird ferner gelöst durch eine Einrichtung mit einem Mikroelektrodenarray der zuvor erläuterten Art und wenigstens einer elektronischen Steuereinrichtung, wobei die elektronische Steuereinrichtung mit einem, mehreren oder allen elektrischen und/oder elektronischen Bauteilen des Mikroelektrodenarrays gekoppelt ist. Insbesondere kann die elektronische Steuereinrichtung mit den Elektroden des Mikroelektrodenarrays, den elektrischen Lichtquellen, weiteren elektrischen und/oder elektronischen Bauteilen und/oder den Tiefenelektroden gekoppelt sein. Die elektronische Steuereinrichtung kann ganz oder teilweise in das Mikroelektrodenarray integriert sein. Auf Grund der Baugröße heutiger elektronischer Steuereinrichtungen ist es vorteilhaft, zumindest einen Teil der elektronischen Steuereinrichtung separat vom Mikroelektrodenarray zu realisieren, z. B. in einem gesonderten Steuergerät, das die vom Mikroelektrodenarray abgegebenen Signale aufnimmt und auswertet und ggf. Steuersignale an die elektrischen Lichtquellen abgibt. Die Steuerung der Elektroden in Abhängigkeit von Sensorsignalen des Mikroelektrodenarrays, z. B. im Rahmen des erwähnten Regelkreises, kann lokal auf dem Mikroelektrodenarray durch einen dort integrierten Teil der elektronischen Steuereinrichtung realisiert werden, oder extern vom Mikroelektrodenarray im separaten Steuergerät.

Die elektronische Steuereinrichtung kann mit einem, mehreren oder allen elektrischen und/oder elektronischen Bauteilen des Mikroelektrodenarrays drahtgebunden und/oder drahtlos gekoppelt sein. Die drahtlose Kopplung hat den Vorteil, dass das Mikroelektrodenarray nach seiner Implantation einfacher handhabbar ist und der Patient geschont wird.

Gemäß einer vorteilhaften Weiterbildung der Erfindung ist die elektronische Steuereinrichtung mit wenigstens einer, mehreren oder allen elektrischen Lichtquellen gekoppelt und zur Steuerung der Lichtabgabe von einer, mehreren oder allen gekoppelten elektrischen Lichtquellen eingerichtet.

Ferner kann vorgesehen sein, dass die elektronische Steuereinrichtung zur Steuerung der Lichtabgabe von einer, mehreren oder allen gekoppelten elektrischen Lichtquellen in Abhängigkeit von elektrischen und/oder elektronischen Sensorbauteilen des Mikroelektrodenarrays, die jeweils ein elektrisches Sensorsignal in Abhängigkeit von wenigstens einer von der elektrischen Lichtquelle oder den elektrischen Lichtquellen beeinflussten physikalischen Größe, die durch das jeweilige Sensorbauteil erfasst ist, eingerichtet ist.

Das Mikroelektrodenarray und die Einrichtung mit einem Mikroelektrodenarray können z.B. für das Mapping von Gehirnfunktionen und/oder zur Analyse epileptogener Zonen verwendet werden. Auch eine Anwendung als Mensch-Maschine-Schnittstelle (HMI) bzw. Gehirn-Maschine-Schnittstelle (BMI) ist vorteil haft.

Die Erfindung wird nachfolgend anhand von Ausführungsbeispielen unter Verwendung von Zeichnungen näher erläutert.

Es zeigen
- Fig. 1:: ein Mikroelektrodenarray in isometrischer Darstellung und
- Fig. 2, 3 und 5:: Ausführungsformen eines Mikroelektrodenarrays in seitlicher Schnittdarstellung und
- Fig. 4 und 6:: die Anwendung eines Mikroelektrodenarrays bei der Aufnahme eines Elektrokortikogrammes.

In den Figuren werden gleiche Bezugszeichen für einander entsprechende Elemente verwendet.

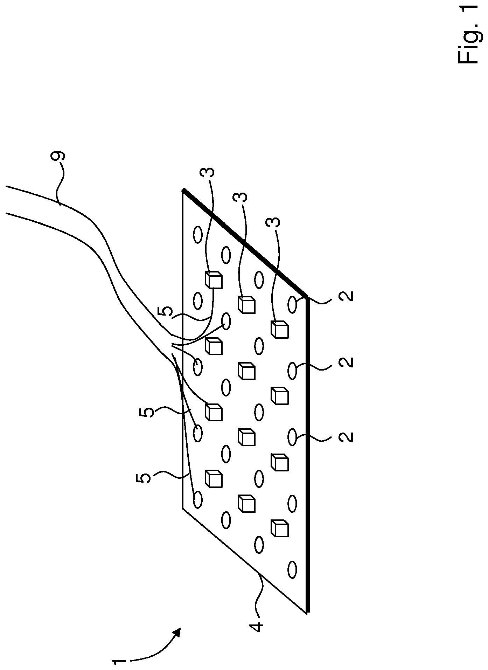

Die Fig. 1 zeigt ein Mikroelektrodenarray 1, das z.B. als Dünnschichtarray mit einem flexiblen, folienartigen dünnen Substrat 4 ausgebildet sein kann. An oder in dem Substrat 4 sind mehrere Elektroden 2, die jeweils durch Kreise dargestellt sind, und mehrere elektrische Lichtquellen 3 in Form von Leuchtdioden, die jeweils in Form von Quadern dargestellt sind, angeordnet. Die Elektroden 2 und die Leuchtdioden 3 sind über elektrische Leitungen 5, von denen aus Übersichtsgründen in der Fig. 1 nur einige Leitungen beispielhaft dargestellt sind, mit einem Verbindungskabel 9 verbunden. Das Verbindungskabel 9 kann z.B. als Flachbandkabel ausgebildet sein. Über das Verbindungskabel 9 werden die elektrischen Signale der Elektroden 2 zu einem Verstärker und einem Messsystem geleitet und zudem die Leuchtdioden 3 mit Strom versorgt.

Das Mikroelektrodenarray 1 kann hinsichtlich der Breite und Länge Maße im Millimeter- oder Zentimeterbereich haben und in unterschiedlichen Formen, die auch von der in Fig. 1 dargestellten Rechteckform abweichen können, ausgebildet sein.

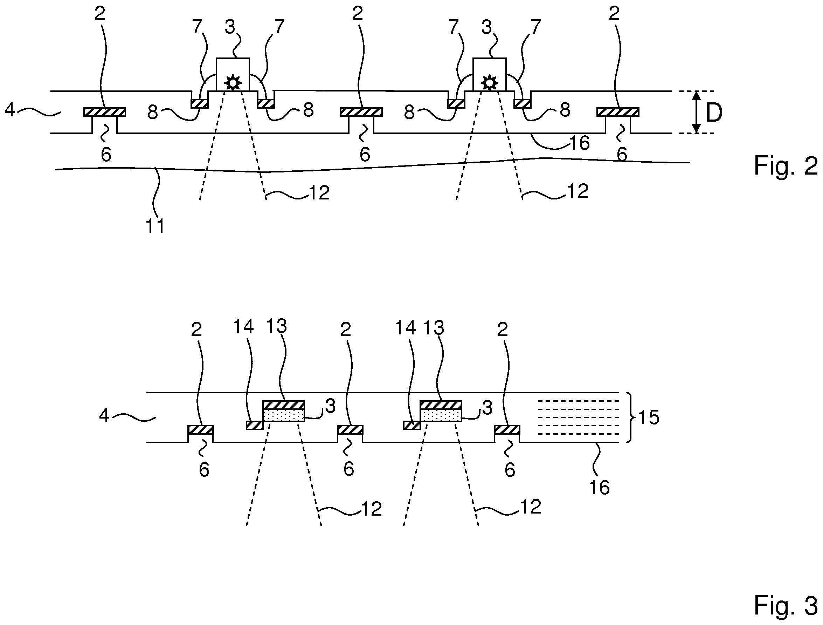

Die Fig. 2 zeigt eine Ausführungsform des Mikroelektrodenarrays 1 im Querschnitt. Die Dicke D des Substrats 4 ist relativ gering im Vergleich zur Breite und Länge. Die Dicke D kann insbesondere im Mikrometer-Bereich liegen. Das als dünne, flexible Folie z.B. aus Parylen, Polyimid, PDMS oder Polyurethan ausgebildete Substrat 4 weist darin eingebrachte Metallstrukturen 2, 8 auf. Die Metallstrukturen 2 bilden in mit Öffnungen 6 versehenen Substratbereichen die Elektroden 2. Die Öffnungen 6 weisen zu einer Sensorseite 16 des Substrats 4 hin, die dazu eingerichtet ist, in Kontakt mit der Hirnoberfläche 11 eines zu untersuchenden Lebewesens gebracht zu werden. Die Metallstrukturen 8 realisieren von den Elektroden 2 separate, d.h. elektrisch davon getrennte, sowie zum biologischen Gewebe bzw. Cortex 11 passivierte Stromversorgungsleitungen der Leuchtdioden 3. Die Leuchtdioden 3 sind auf der der Hirnoberfläche 11 abgewandten Seite des Substrats 4 auf dieses Substrat 4 aufgebracht oder in das Substrat 4 integriert, z. B. nach Art einer Sandwichstruktur. Die Leuchtdioden 3 sind entweder direkt oder mit elektrischen Verbindungen 7, z.B. in Form von Bonds, mit den Stromversorgungsleitungen 8 elektrisch verbunden. Die Leuchtdioden 3 strahlen ihr Licht 12 durch das optisch hinreichend transparente Substrat 4 in Richtung zur Hirnoberfläche 11 ab und stimulieren dadurch die darin vorhandenen Nervenzellen. Die Nervenzellen können z.B. mit Kanalrhodopsinen lichtsensitiv gemacht werden. Der Pfad des Lichts 12 kann dabei auch durch die Metallstrukturen 2, 8 oder durch zusätzliche Elemente, wie z.B. refraktive oder reflektive optische Elemente, die im Mikroelektrodenarray 1 vorhanden sind, beeinflusst werden.

Die Fig. 3 zeigt eine weitere Ausführungsform eines Mikroelektrodenarrays in Querschnittsdarstellung. Gemäß Fig. 3 sind die Leuchtdioden 3 in Form von in das Substrat 4 integrierten Dünnschicht-LEDs ausgebildet, insbesondere als organische LEDs. Das Substrat 4 kann in diesem Fall vorteilhaft als Mehrschichtstruktur 15 aus mehreren Lagen hergestellt sein. Die Leuchtdioden 3 sind dann direkt in die Mehrschichtstruktur 15 des Substrats 4 mikrostrukturiert eingebracht. Sie werden durch Metalllayer 13, 14 in der Mehrschichtstruktur 15 kontaktiert, um die Stromversorgung zu gewährleisten. Die Elektroden 2 sind wiederum über Öffnungen 6 zur Sensorseite 16 hin geöffnet. Alle anderen Strukturen sind elektrisch und biologisch passiviert.

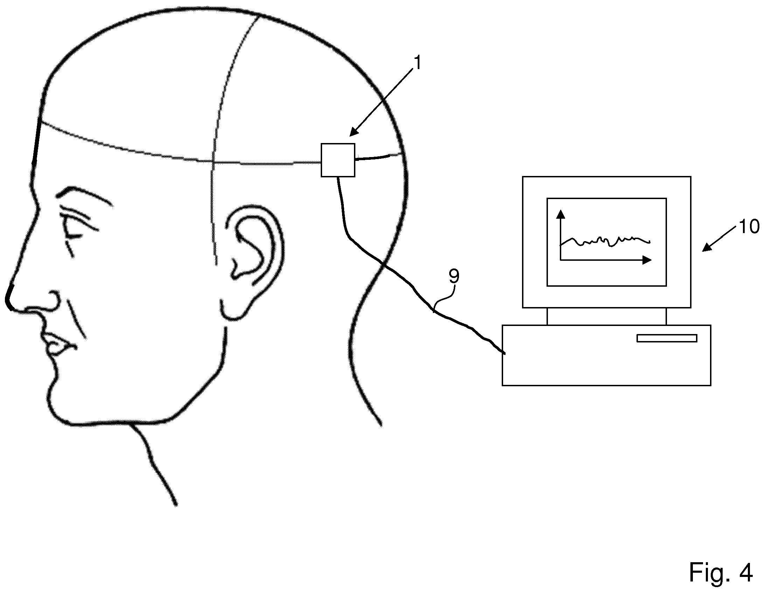

Die Fig. 4 zeigt eine Anwendung des erfindungsgemäßen Mikroelektrodenarrays 1 bei der Aufnahme eines Elektrokortikogramms eines Menschen. Das Mikroelektrodenarray 1 ist über das Verbindungskabel 9 mit einer elektronischen Einrichtung 10 verbunden, die insbesondere einen Verstärker für die Elektrodensignale und ein Datenerfassungssystem beinhaltet. Die aufgenommenen Daten können z.B. auf einem Bildschirm dargestellt werden.

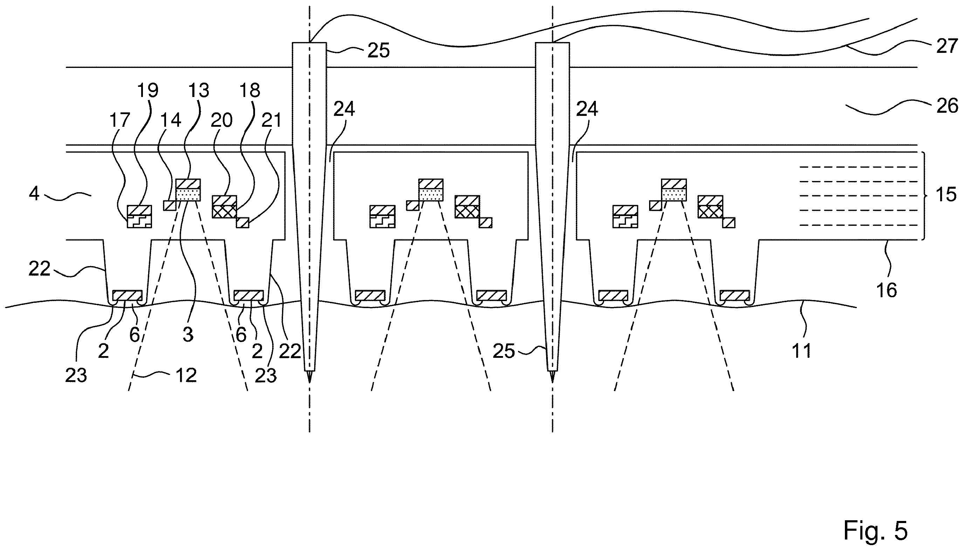

Die Fig. 5 zeigt eine weitere Ausführungsform eines Mikroelektrodenarrays in Querschnittsdarstellung. Gemäß Fig. 5 sind die Elektroden 2 in erhabenen, aus dem Substratmaterial hergestellten Säulenstrukturen 22 zur Annäherung an die Hirnoberfläche 11 eingebettet und wiederum über Öffnungen 6 zur Sensorseite 16 hin geöffnet. Mikrostrukturierte, umlaufende Dichtlippen 23 begrenzen die aktiven Elektrodenflächen der Elektroden 2 lateral. Die Leuchtdioden 3 sind in Form von in das Substrat 4 integrierten Dünnschicht-LEDs ausgebildet, insbesondere als phosphoreszierende organische Leuchtdioden, und sind direkt in die Mehrschichtstruktur 15 des Substrats 4 mikrostrukturiert eingebracht. Sie werden durch die Metallstrukturen 13, 14 in der Mehrschichtstruktur 15 kontaktiert, um die Stromversorgung zu gewährleisten und strahlen ihr Licht 12 durch das optisch hinreichend transparente Substrat 4 in Richtung zur Hirnoberfläche 11 ab. Die Metallstrukturen 13, 14, 19, 20, 21 realisieren von den Elektroden 2 separate, d.h. elektrisch davon getrennte, sowie zum biologischen Gewebe bzw. Cortex 11 passivierte, elektrische Verbindungsleitungen. Dabei bilden die Metallstrukturen 19 die Stromversorgungsleitung der beispielsweise als Mäander ausgeführten Temperatursensoren 17 und die Metallstrukturen 20, 21 die elektrische Kontaktierung der Photodioden 18. Temperatursensoren 17 sowie Photodioden 18 sind im Substrat, z.B. nach Art einer Sandwichstruktur, eingebettet und somit elektrisch und biologisch passiviert. In dem Substrat 4 sind Durchgangsöffnungen 24 angeordnet, die dazu geeignet sind die Diffusion von pharmakologischen Substanzen ins Gewebe, für z.B. optogenetische Anwendungen, zu erlauben und/oder penetrierende Tiefenelektroden 25 in das kortikale Gewebe einzubringen. Die Tiefenelektroden 25 sind in einem angepassten Tiefenelektrodenarray 26 in definiertem Abstand ortsfest integriert, wobei die kabelgebundenen, z.B. als Flachbandkabel ausgeführten, elektrischen Verbindungsleitungen 27 auf der von der Sensorseite 16 abgewandten Seite weggeführt werden.

Die Fig. 5 zeigt nebeneinander drei der zuvor beschriebenen Anordnungen in dem Substrat 4, die gleich aufgebaut sind und von denen daher wegen der besseren Übersichtlichkeit nur eine Anordnung vollständig mit Bezugszeichen versehen wurde.

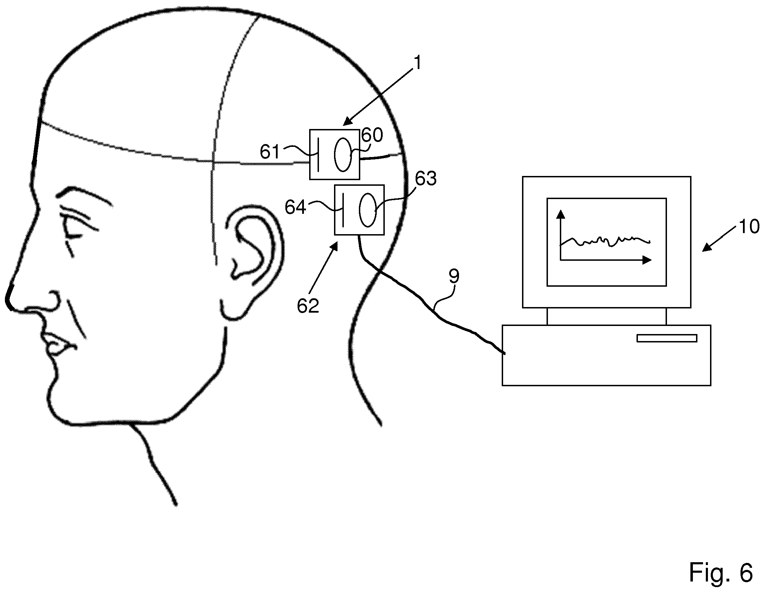

Die Fig. 6 zeigt eine Anwendung des erfindungsgemäßen Mikroelektrodenarrays 1 bei der Aufnahme eines Elektrokortikogramms eines Menschen. Im Unterschied zur Fig. 4 ist das Mikroelektrodenarray 1 drahtlos mit einer elektronischen Steuereinrichtung 10 verbunden, die insbesondere einen Verstärker für die Elektrodensignale und ein Datenerfassungssystem beinhaltet. Das z.B. unter dem Skalp des Patienten implantierte Mikroelektrodenarray 1 weist eine Energieempfangsspule 60 sowie eine Antenne 61 für die bidirektionale Datenübertragung zwischen dem Mikroelektrodenarray 1 und der elektronischen Steuereinrichtung 10 auf. Es ist auch möglich, die Energieempfangsspule 60 zugleich als Antenne zu verwenden, so dass keine separate Antenne 61 erforderlich ist.

Die elektronische Steuereinrichtung 10 ist hierfür über ein Kabel 9 mit einen Satellitenpad 62 verbunden, in dem eine Energiesendespule 63 und eine Antenne 64 angeordnet sind. Über die Energiesendespule 63 wird über ein Hochfrequenzsignal elektrisch Energie in die Energieempfangsspule 60 eingespeist, so dass das Mikroelektrodenarray 1 drahtlos mit der für seinen Betrieb erforderlichen elektrischen Energie versorgt wird. Über die Antennen 61, 63 erfolgt eine bidirektionale Datenkommunikation zwischen der elektronischen Steuereinrichtung 10 und dem Mikroelektrodenarray 1.

## Patentansprüche

1. Mikroelektrodenarray (1) mit einem Substrat (4) und mit einer Vielzahl von Elektroden (2) zur elektrischen Messung von Hirnströmen und mit im Mikroelektrodenarray (1) integrierter optischer Stimulationseinheit zur Stimulation von Hirnbereichen (11) mit optischen Signalen, wobei die Stimulationseinheit mehrere elektrische Lichtquellen (3) aufweist und die elektrischen Lichtquellen (3) nach einem festen, vorgegebenen Schema relativ zu den Elektroden (2) angeordnet und über die flächige Ausdehnung des Substrats (4) verteilt sind, so dass die über die Elektroden (2) aufgenommene Stimulation der Neuronen mittels der elektrischen Lichtquellen (3) den einzelnen Stimulationssignalen eindeutig zugeordnet werden kann, so dass eine eindeutige Korrelation bestimmt werden kann, und wobei das Mikroelektrodenarray eine Sensorseite (16) aufweist, die dazu eingerichtet ist, in Kontakt mit der Hirnoberfläche (11) eines zu untersuchenden Lebewesens gebracht zu werden, **dadurch gekennzeichnet, dass** eine, mehrere oder alle elektrischen Lichtquellen (3) in größerem Abstand von der Sensorseite (16) angeordnet sind als die Elektroden (2), wobei eine, mehrere oder alle Elektroden (2) in das Material des Substrats (4) integriert sind, indem die Elektroden (2) im Substrat (4) eingebettet sind, und das Substrat (4) an der Sensorseite (16) zu den integrierten Elektroden (2) führende Öffnungen (6) aufweist.

2. Mikroelektrodenarray nach Anspruch 1, **dadurch gekennzeichnet, dass** das Mikroelektrodenarray (1) ein folienartiges, dünnes Substrat (4) aufweist, wobei die Elektroden (2) über die flächige Ausdehnung des Substrats (4) verteilt an der Oberfläche des Substrats (4) oder in dem Substrat (4) angeordnet sind.

3. Mikroelektrodenarray nach einem der vorhergehenden Ansprüche, **dadurch gekennzeichnet, dass** das Mikroelektrodenarray (1) ein folienartiges, dünnes Substrat (4) aufweist, wobei eine Vielzahl von elektrischen Lichtquellen (3) der Stimulationseinheit über die flächige Ausdehnung des Substrats (4) verteilt an der Oberfläche des Substrats (4) oder in dem Substrat (4) angeordnet sind.

4. Mikroelektrodenarray nach einem der vorhergehenden Ansprüche, **dadurch gekennzeichnet, dass** das Substrat (4) ein zumindest teilweise transparentes Folienmaterial aufweist oder daraus besteht.

5. Mikroelektrodenarray nach einem der vorhergehenden Ansprüche, **dadurch gekennzeichnet, dass** auf der Oberfläche des Substrats (4) oder in dem Substrat (4) elektrisch leitende Strukturen gebildet sind, die Elektroden (2), elektrische Verbindungsleitungen zu den Elektroden (2) und/oder elektrische Verbindungsleitungen (8, 13, 14) zu der elektrischen Lichtquelle (3) bzw. den elektrischen Lichtquellen (3) bilden.

6. Mikroelektrodenarray nach einem der vorhergehenden Ansprüche, **dadurch gekennzeichnet, dass** eine, mehrere oder alle elektrischen Lichtquellen (3) als SMD-Bauelemente, als Dies oder als Dünnschichtelemente auf dem Substrat (4) oder innerhalb des Substrats (4) angeordnet sind.

7. Mikroelektrodenarray nach einem der vorhergehenden Ansprüche, **dadurch gekennzeichnet, dass** eine, mehrere oder alle elektrischen Lichtquellen (3) als Leuchtdioden ausgebildet sind, insbesondere als anorganische oder organische Leuchtdioden (OLEDs).

8. Mikroelektrodenarray nach einem der vorhergehenden Ansprüche, **dadurch gekennzeichnet, dass** zumindest die Sensorseite (16) des Mikroelektrodenarrays (1) elektrisch und biologisch passiviert ist.

9. Mikroelektrodenarray nach einem der vorhergehenden Ansprüche, **dadurch gekennzeichnet, dass** eine, mehrere oder alle Elektroden (2) als ECoG-Elektroden ausgebildet sind.

10. Mikroelektrodenarray nach einem der vorhergehenden Ansprüche, **dadurch gekennzeichnet, dass** das Mikroelektrodenarray (1) eine oder mehrere Stimulationselektroden zur Stimulation von Hirnbereichen mit elektrischen Signalen aufweist.

11. Mikroelektrodenarray nach Anspruch 10, **dadurch gekennzeichnet, dass** eine oder mehrere Elektroden (2) zur elektrischen Messung von Hirnströmen zugleich Stimulationselektroden zur Stimulation von Hirnbereichen mit elektrischen Signalen sind.

12. Mikroelektrodenarray nach einem der vorhergehenden Ansprüche, **dadurch gekennzeichnet, dass** in relativer Nähe zu einer, mehreren oder allen elektrischen Lichtquellen (3) jeweils ein oder mehrere weitere elektrische und/oder elektronische Bauteile, insbesondere Sensorbauteile (17, 18), angeordnet sind.

13. Mikroelektrodenarray nach Anspruch 12, **dadurch gekennzeichnet, dass** in relativer Nähe zu einer, mehreren oder allen elektrischen Lichtquellen (3) jeweils ein oder mehrere weitere elektrische und/oder elektronische Sensorbauteile (17, 18), die jeweils ein elektrisches Sensorsignal abgeben, derart angeordnet sind, dass wenigstens eine von der elektrischen Lichtquelle (3) oder den elektrischen Lichtquellen (3) beeinflusste physikalische Größe durch das jeweilige Sensorbauteil (17, 18) erfassbar ist.

14. Mikroelektrodenarray nach Anspruch 13, **dadurch gekennzeichnet, dass** eine, mehrere oder alle elektrischen Lichtquellen (3) der jeweiligen Lichtquelle zugeordnete ein oder mehrere Sensorbauteile (17, 18) aufweisen, die jeweils ein elektrisches Sensorsignal abgeben, das der beeinflussten physikalischen Größe einer bestimmten Lichtquelle zuordenbar ist.

15. Mikroelektrodenarray nach einem der Ansprüche 13 bis 14, **dadurch gekennzeichnet, dass** einer, mehrere oder alle der Sensorbauteile (17, 18) als Temperatursensoren (17) ausgebildet sind.

16. Mikroelektrodenarray nach einem der Ansprüche 13 bis 15, **dadurch gekennzeichnet, dass** einer, mehrere oder alle der Sensorbauteile (17, 18) als lichtempfindliche Sensoren (18) ausgebildet sind.

17. Mikroelektrodenarray nach einem der Ansprüche 12 bis 16, **dadurch gekennzeichnet, dass** die weiteren elektrischen und/oder elektronischen Bauteile nach einem festen, vorgegebenen Schema relativ zu den Lichtquellen (3) angeordnet und über die flächige Ausdehnung des Substrats (4) verteilt an der Oberfläche des Substrats (4) oder in dem Substrat (4) angeordnet sind.

18. Mikroelektrodenarray nach einem der Ansprüche 12 bis 17, **dadurch gekennzeichnet, dass** auf der Oberfläche des Substrats (4) oder in dem Substrat (4) elektrisch leitende Strukturen gebildet sind, die elektrische Verbindungsleitungen (19, 20, 21) zu dem weiteren elektrischen und/oder elektronischen Bauteil oder zu den weiteren elektrischen und/oder elektronischen Bauteilen bilden.

19. Mikroelektrodenarray nach einem der Ansprüche 12 bis 18, **dadurch gekennzeichnet, dass** eines, mehrere oder alle weiteren elektrischen und/oder elektronischen Bauteile als SMD-Bauelemente, als Dies oder als Dünnschichtelemente auf dem Substrat (4) oder innerhalb des Substrats (4) angeordnet sind.

20. Mikroelektrodenarray nach einem der vorhergehenden Ansprüche, **dadurch gekennzeichnet, dass** eine, mehrere oder alle elektrischen Lichtquellen (3) als phosphoreszierende organische Leuchtdioden (PHOLEDs) ausgebildet sind.

21. Mikroelektrodenarray nach einem der vorhergehenden Ansprüche, **dadurch gekennzeichnet, dass** das Mikroelektrodenarray (1) eine Empfangsvorrichtung zum drahtlosen Empfang von auf das Mikroelektrodenarray (1) abgestrahlten elektromagnetischen Wellen aufweist und das Mikroelektrodenarray (1) mit der für seinen Betrieb notwendigen elektrischen Energie über die Empfangsvorrichtung drahtlos versorgbar ist und/oder ein Akkumulator des Mikroelektrodenarrays (1) über die Empfangsvorrichtung drahtlos aufladbar ist.

22. Mikroelektrodenarray nach einem der vorhergehenden Ansprüche, **dadurch gekennzeichnet, dass** das Mikroelektrodenarray (1) eine drahtlos arbeitende Signalübertragungseinrichtung aufweist, die zum drahtlosen Übertragen von Signalen, insbesondere in Form von Daten, von dem Mikroelektrodenarray (1) zu einer Signalempfangseinrichtung und/oder zum drahtlosen Übertragen von Signalen, insbesondere in Form von Daten, von einer Signalsendeeinrichtung zu dem Mikroelektrodenarray (1) eingerichtet ist.

23. Mikroelektrodenarray (1) nach einem der vorhergehenden Ansprüche, **dadurch gekennzeichnet, dass** eine, mehrere oder alle Elektroden (2) in einer jeweils erhabenen Säulenstruktur (22) integriert sind, welche die einzelnen Elektroden (2) auf der Sensorseite (16) von der Substratebene beabstandet.

24. Mikroelektrodenarray (1) nach Anspruch 23, **dadurch gekennzeichnet, dass** eine, mehrere oder alle Säulenstrukturen (19) aus dem Substratmaterial gefertigt sind.

25. Mikroelektrodenarray (1) nach einem der vorhergehenden Ansprüche, **dadurch gekennzeichnet, dass** eine, mehrere oder alle Säulenstrukturen (19) eine jeweils umlaufende Dichtlippe (23) aufweisen, die die aktive Elektrodenfläche der in die Säulenstruktur (22) integrierten Elektrode (2) lateral begrenzt.

26. Mikroelektrodenarray (1) nach einem der vorhergehenden Ansprüche, **dadurch gekennzeichnet, dass** Durchgangsöffnungen (24) im Substrat (4) angeordnet sind, die dazu geeignet sind die Diffusion von pharmakologischen Substanzen ins Gewebe zu erlauben und/oder eine oder mehrere penetrierende Tiefenelektroden (25) in das Gewebe einzubringen.

27. Mikroelektrodenarray nach Anspruch 26, **dadurch gekennzeichnet, dass** mehrere oder alle Durchgangsöffnungen (24) nach einem festen, vorgegebenen Muster über die flächige Ausdehnung des Substrats (4) verteilt angeordnet sind.

28. Mikroelektrodenarray (1) nach Anspruch 26 oder 27, **dadurch gekennzeichnet, dass** eine oder mehrere Tiefenelektroden (25) oder wenigstens ein Tiefenelektrodenarray (26) zur simultanen elektrischen Messung von Hirnströmen tieferer Hirnbereiche und/oder der elektrischen sowie optischen Stimulation von tieferen Hirnbereichen in die Durchgangsöffnungen (24) eingeführt sind.

29. Einrichtung mit einem Mikroelektrodenarray nach einem der vorhergehenden Ansprüche und wenigstens einer elektronischen Steuereinrichtung, **dadurch gekennzeichnet, dass** die elektronische Steuereinrichtung mit einem, mehreren oder allen elektrischen und/oder elektronischen Bauteilen des Mikroelektrodenarrays (1) gekoppelt ist.

30. Einrichtung nach Anspruch 29, **dadurch gekennzeichnet, dass** die elektronische Steuereinrichtung mit einem, mehreren oder allen elektrischen und/oder elektronischen Bauteilen des Mikroelektrodenarrays (1) drahtgebunden und/oder drahtlos gekoppelt ist.

31. Einrichtung nach einem der Ansprüche 29 bis 30, **dadurch gekennzeichnet, dass** die elektronische Steuereinrichtung wenigstens mit einer, mehreren oder allen elektrischen Lichtquellen (3) gekoppelt ist und zur Steuerung der Lichtabgabe von einer, mehreren oder allen gekoppelten elektrischen Lichtquellen (3) eingerichtet ist.

32. Einrichtung nach Anspruch 30, **dadurch gekennzeichnet, dass** die elektronische Steuereinrichtung zur Steuerung der Lichtabgabe von einer, mehreren oder allen gekoppelten elektrischen Lichtquellen (3) in Abhängigkeit von elektrischen und/oder elektronischen Sensorbauteilen des Mikroelektrodenarrays (1), die jeweils ein elektrisches Sensorsignal in Abhängigkeit von wenigstens einer von der elektrischen Lichtquelle (3) oder den elektrischen Lichtquellen (3) beeinflussten physikalischen Größe, die durch das jeweilige Sensorbauteil erfasst ist, eingerichtet ist.

## Claims

1. Microelectrode array (1) comprising a substrate (4) and comprising a multiplicity of electrodes (2) for electrically measuring brain waves and comprising an optical stimulation unit integrated in the microelectrode array (1) for stimulating brain regions (11) with optical signals, wherein the stimulation unit has a plurality of electrical light sources (3)and the electrical light sources (3) are arranged according to a fixed, predefined scheme relative to the electrodes (2) and are distributed over the areal extent of the substrate (4), such that the simulation-recorded via the electrodes (2) - of the neurons by means of the electrical light sources (3) can be unambiguously assigned to the individual stimulation signals, such that an unambiguous correlation can be determined, and wherein the microelectrode array has a sensor side (16), which is designed to be brought into contact with the brain surface (11) of a living being to be examined, **characterized in that** one, a plurality or all of the electrical light sources (3) are arranged at a greater distance from the sensor side (16) than the electrodes (2) wherein one, a plurality or all of the electrodes (2) is/are integrated into the material of the substrate (4) by virtue of the foot that the electrodes (2) are embedded in the substrate (4), and the substrate (4), on the sensor side (16), has openings (6) leading to the integrated electrodes (2), in the microelectrode array (1).

2. Microelectrode array according to Claim 1, **characterized in that** the microelectrode array (1) has a filmlike, thin substrate (4), wherein the electrodes (2) are arranged in a manner distributed over the areal extent of the substrate (4) on the surface of the substrate (4) or in the substrate (4).

3. Microelectrode array according to either of the preceding claims, **characterized in that** the microelectrode array (1) has a filmlike, thin substrate (4), wherein a multiplicity of electrical light sources (3) of the stimulation unit are arranged in a manner distributed over the areal extent of the substrate (4) on the surface of the substrate (4) or in the substrate (4).

4. Microelectrode array according to any of the preceding claims, **characterized in that** the substrate (4) comprises or consists of an at least partly transparent film material.

5. Microelectrode array according to any of the preceding claims, **characterized in that** electrically conductive structures are formed on the surface of the substrate (4) or in the substrate (4), said electrically conductive structures forming electrodes (2), electrical connection lines to the electrodes (2) and/or electrical connection lines (8, 13, 14) to the electrical light source (3) or the electrical light sources (3).

6. Microelectrode array according to any of the preceding claims, **characterized in that** one, a plurality or all of the electrical light sources (3) is/are arranged as SMD components, as dies or as thin-film elements on the substrate (4) or within the substrate (4).

7. Microelectrode array according to any of the preceding claims, **characterized in that** one, a plurality or all of the electrical light sources (3) is/are embodied as light emitting diodes, in particular as inorganic or organic light emitting diodes (OLEDs).

8. Microelectrode array according to any of the preceding claims, **characterized in that** at least the sensor side (16) of the microelectrode array (1) is electrically and biologically passivated.

9. Microelectrode array according to any of the preceding claims, **characterized in that** one, a plurality or all of the electrodes (2) is/are embodied as ECoG electrodes.

10. Microelectrode array according to any of the preceding claims, **characterized in that** the microelectrode array (1) has one or a plurality of stimulation electrodes for stimulating brain regions with electrical signals.

11. Microelectrode array according to Claim 10, **characterized in that** one or a plurality of electrodes (2) for electrically measuring brain waves is/are simultaneously stimulation electrodes for stimulating brain regions with electrical signals.

12. Microelectrode array according to any of the preceding claims, **characterized in that** in each case one or a plurality of further electrical and/or electronic components, in particular sensor components (17, 18), is/are arranged in relative proximity to one, a plurality or all of the electrical light sources (3).

13. Microelectrode array according to Claim 12, **characterized in that** in each case one or a plurality of further electrical and/or electronic sensor components (17, 18), each of which outputs an electrical sensor signal, is/are arranged in relative proximity to one, a plurality or all of the electrical light sources (3) in such a way that at least one physical variable influenced by the electrical light source (3) or the electrical light sources (3) is detectable by the respective sensor component (17, 18).

14. Microelectrode array according to Claim 13, **characterized in that** one, a plurality or all of the electrical light sources (3) have one or a plurality of sensor components (17, 18) assigned to the respective light source, each of which sensor components outputs an electrical sensor signal which is assignable to the influenced physical variable of a specific light source.

15. Microelectrode array according to either of Claims 13 and 14, **characterized in that** one, a plurality or all of the sensor components (17, 18) is/are embodied as temperature sensors (17).

16. Microelectrode array according to any of Claims 13 to 15, **characterized in that** one, a plurality or all of the sensor components (17, 18) is/are embodied as light-sensitive sensors (18).

17. Microelectrode array according to any of Claims 12 to 16, **characterized in that** the further electrical and/or electronic components are arranged according to a fixed, predefined scheme relative to the light sources (3) and are arranged in a manner distributed over the areal extent of the substrate (4) on the surface of the substrate (4) or in the substrate (4).

18. Microelectrode array according to any of Claims 12 to 17, **characterized in that** electrically conductive structures are formed on the surface of the substrate (4) or in the substrate (4), said electrically conductive structures forming electrical connection lines (19, 20, 21) to the further electrical and/or electronic component or to the further electrical and/or electronic components.

19. Microelectrode array according to any of Claims 12 to 18, **characterized in that** one, a plurality or all of the further electrical and/or electronic components are arranged as SMD components, as dies or as thin-film elements on the substrate (4) or within the substrate (4).

20. Microelectrode array according to any of the preceding claims, **characterized in that** one, a plurality or all of the electrical light sources (3) is/are embodied as phosphorescent organic light emitting diodes (PHOLEDs).

21. Microelectrode array according to any of the preceding claims, **characterized in that** the microelectrode array (1) has a receiving apparatus for wirelessly receiving electromagnetic waves emitted onto the microelectrode array (1), and the microelectrode array (1) is wirelessly suppliable with the electrical energy required for its operation via the receiving apparatus and/or a rechargeable battery of the microelectrode array (1) is wirelessly chargeable via the receiving apparatus.

22. Microelectrode array according to any of the preceding claims, **characterized in that** the microelectrode array (1) has a wirelessly operating signal transfer device designed for wirelessly transferring signals, in particular in the form of data, from the microelectrode array (1) to a signal receiving device and/or for wirelessly transferring signals, in particular in the form of data, from a signal transmitting device to the microelectrode array (1).

23. Microelectrode array (1) according to any of the preceding claims, **characterized in that** one, a plurality or all of the electrodes (2) is/are integrated in a respectively elevated column structure (22) which spaces apart the individual electrodes (2) on the sensor side (16) from the substrate plane.

24. Microelectrode array (1) according to Claim 23, **characterized in that** one, a plurality or all of the column structures (19) is/are produced from the substrate material.

25. Microelectrode array (1) according to any of the preceding claims, **characterized in that** one, a plurality or all of the column structures (19) has/have a respectively circumferential sealing lip (23) which laterally delimits the active electrode area of the electrode (2) integrated into the column structure (22).

26. Microelectrode array (1) according to any of the preceding claims, **characterized in that** through openings (24) are arranged in the substrate (4), said through openings being suitable for allowing the diffusion of pharmacological substances into the tissue and/or for introducing one or a plurality of penetrating depth electrodes (25) into the tissue.

27. Microelectrode array according to Claim 26, **characterized in that** a plurality or all of the through openings (24) are arranged in a manner distributed over the areal extent of the substrate (4) according to a fixed, predefined pattern.

28. Microelectrode array (1) according to Claim 26 or 27, **characterized in that** one or a plurality of depth electrodes (25) or at least one depth electrode array (26) for simultaneously electrically measuring brain waves of deeper brain regions and/or electrically and optically stimulating deeper brain regions is/are inserted into the through openings (24).

29. Device comprising a microelectrode array according to any of the preceding claims and at least one electronic control device, **characterized in that** the electronic control device is coupled to one, a plurality or all of the electrical and/or electronic components of the microelectrode array (1) .

30. Device according to Claim 29, **characterized in that** the electronic control device is coupled to one, a plurality or all of the electrical and/or electronic components of the microelectrode array (1) in a wired fashion and/or wirelessly.

31. Device according to either of Claims 29 and 30, **characterized in that** the electronic control device is coupled at least to one, a plurality or all of the electrical light sources (3) and is designed for controlling the light emission from one, a plurality or all of the coupled electrical light sources (3).

32. Device according to Claim 30, **characterized in that** the electronic control device is designed for controlling the light emission from one, a plurality or all of the coupled electrical light sources (3) depending on electrical and/or electronic sensor components of the microelectrode array (1), each of which an electrical sensor signal depending on at least one physical variable which is influenced by the electrical light source (3) or the electrical light sources (3) and which is detected by the respective sensor component.

## Revendications

1. Réseau de microélectrodes (1) comportant un substrat (4) et une pluralité d'électrodes (2) pour la mesure électrique de flux cérébraux et comportant une unité de stimulation optique intégrée au réseau de microélectrodes (1) pour la stimulation de zones cérébrales (11) au moyen de signaux optiques, dans lequel l'unité de stimulation comporte plusieurs sources de lumière électriques (3) et les sources de lumière électriques (3) sont disposées par rapport aux électrodes (2) selon un schéma fixe prédéterminé et sont réparties sur l'étendue plane du substrat (4), de manière à ce la stimulation des neurones par les sources de lumière électriques (3), acquise par l'intermédiaire des électrodes (2), puisse être associée sans ambiguïté aux signaux de stimulation individuels afin qu'une corrélation non ambiguë puisse être déterminée, et dans lequel le réseau de microélectrodes comporte un côté capteur (16) conçu pour être mis en contact avec la surface du cerveau (11) d'un être vivant à examiner, **caractérisé en ce que** l'une, plusieurs ou la totalité des sources de lumière électriques (3) sont disposées à une plus grande distance du côté capteur (16) que les électrodes (2), dans lequel l'une, plusieurs ou la totalité des électrodes (2) sont intégrées dans le matériau du substrat (4) en incorporant les électrodes (2) dans le substrat (4), et le substrat (4) présente, côté capteur (16), des ouvertures (6) menant vers les électrodes intégrées (2).

2. Réseau de microélectrodes selon la revendication 1, **caractérisé en ce que** le réseau de microélectrodes (1) comporte un substrat mince (4) de type film, dans lequel les électrodes (2) sont disposées de manière répartie sur l'étendue plane du substrat (4) à la surface du substrat (4) ou dans le substrat (4).

3. Réseau de microélectrodes selon l'une des revendications précédentes, **caractérisé en ce que** le réseau de microélectrodes (1) comprend un substrat mince de type film (4), dans lequel une pluralité de sources de lumière électriques (3) de l'unité de stimulation sont disposées de manière répartie sur l'étendue plane du substrat (4) à la surface du substrat (4) ou dans le substrat (4).

4. Réseau de microélectrodes selon l'une des revendications précédentes, **caractérisé en ce que** le substrat (4) comporte ou consiste en un matériau de type film au moins partiellement transparent.

5. Réseau de microélectrodes selon l'une des revendications précédentes, **caractérisé en ce que** des structures électriquement conductrices sont formées sur la surface du substrat (4) ou dans le substrat (4), lesquelles structures forment des électrodes (2), des lignes de connexion électriques aux électrodes (2) et/ou des lignes de connexion électriques (8, 13, 14) à la source de lumière électrique (3) ou aux sources de lumière électriques (3).

6. Réseau de microélectrodes selon l'une des revendications précédentes, **caractérisé en ce que** l'une, plusieurs ou la totalité des sources de lumière électriques (3) sont disposées sous la forme de composants SMD, de matrices ou d'éléments à couches minces sur le substrat (4) ou à l'intérieur du substrat (4) .

7. Réseau de microélectrodes selon l'une des revendications précédentes, **caractérisé en ce que** l'une, plusieurs ou la totalité des sources de lumière électriques (3) sont réalisées sous la forme de diodes électroluminescentes, notamment de diodes électroluminescentes inorganiques ou organiques (OLEDs).

8. Réseau de microélectrodes selon l'une des revendications précédentes, **caractérisé en ce qu'**au moins le côté capteur (16) du réseau de microélectrodes (1) est passivé électriquement et biologiquement.

9. Réseau de microélectrodes selon l'une des revendications précédentes, **caractérisé en ce que** l'une, plusieurs ou la totalité des électrodes (2) sont réalisées sous la forme d'électrodes d'ECoG.

10. Réseau de microélectrodes selon l'une des revendications précédentes, **caractérisé en ce que** le réseau de microélectrodes (1) comporte une ou plusieurs électrodes de stimulation pour la stimulation de zones cérébrales par des signaux électriques.

11. Réseau de microélectrodes selon la revendication 10, **caractérisé en ce qu'**une ou plusieurs électrodes (2) destinées à la mesure électrique de flux cérébraux sont en même temps des électrodes de stimulation destinées à la stimulation de zones cérébrales par des signaux électriques.

12. Réseau de microélectrodes selon l'une des revendications précédentes, **caractérisé en ce qu'**un ou plusieurs autres composants électriques et/ou électroniques, en particulier des composants de capteurs (17, 18), sont disposés relativement près de l'une, de plusieurs ou de la totalité des sources de lumière électriques (3).

13. Réseau de microélectrodes selon la revendication 12, **caractérisé en ce qu'**un ou plusieurs autres composants de capteurs électriques et/ou électroniques (17, 18), qui délivrent respectivement un signal de capteur électrique, sont disposés relativement près de l'une, de plusieurs ou de la totalité de sources de lumière électriques (3), de manière à ce qu'au moins une grandeur physique influencée par l'une de la source de lumière électrique (3) ou des sources de lumière électriques (3) puisse être détectée par le composant de capteur (17, 18) respectif.

14. Réseau de microélectrodes selon la revendication 13, **caractérisé en ce que** l'une, plusieurs ou la totalité des sources de lumière électriques (3) comportent un ou plusieurs composants de capteurs (17, 18) associés à la source de lumière respective, qui délivrent respectivement un signal de capteur électrique pouvant être associé à la grandeur physique influencée d'une source de lumière déterminée.

15. Réseau de microélectrodes selon l'une des revendications 13 à 14, **caractérisé en ce que** l'un, plusieurs ou la totalité des composants de capteurs (17, 18) sont réalisés sous la forme de capteurs de température (17).

16. Réseau de microélectrodes selon l'une des revendications 13 à 15, **caractérisé en ce que** l'un, plusieurs ou la totalité des composants de capteurs (17, 18) sont réalisés sous la forme de capteurs photosensibles (18).

17. Réseau de microélectrodes selon l'une des revendications 12 à 16, **caractérisé en ce que** les autres composants électriques et/ou électroniques sont disposés selon un schéma fixe prédéterminé par rapport aux sources de lumière (3) et sont disposés de manière répartie sur l'étendue plane du substrat (4) à la surface du substrat (4) ou dans le substrat (4).

18. Réseau de microélectrodes selon l'une des revendications 12 à 17, **caractérisé en ce que** des structures électriquement conductrices sont formées sur la surface du substrat (4) ou dans le substrat (4), lesquelles structures forment des lignes de connexion électriques (19, 20, 21) à l'autre composant électrique et/ou électronique ou aux autres composants électriques et/ou électroniques.

19. Réseau de microélectrodes selon l'une des revendications 12 à 18, **caractérisé en ce que** l'un, plusieurs ou la totalité des autres composants électriques et/ou électroniques sont disposés sur le substrat (4) ou à l'intérieur du substrat (4) sous la forme de composants SMD, de matrices ou d'éléments à couches minces.

20. Réseau de microélectrodes selon l'une des revendications précédentes, **caractérisé en ce que** l'une, plusieurs ou la totalité des sources de lumière électriques (3) sont réalisées sous la forme de diodes électroluminescentes organiques phosphorescentes (PHOLEDs).

21. Réseau de microélectrodes selon l'une des revendications précédentes, **caractérisé en ce que** le réseau de microélectrodes (1) comporte un dispositif de réception pour la réception sans fil d'ondes électromagnétiques rayonnées vers le réseau de microélectrodes (1), et **en ce que** le réseau de microélectrodes (1) peut être alimenté sans fil en l'énergie électrique nécessaire à son fonctionnement par l'intermédiaire du dispositif de réception et/ou **en ce qu'**un accumulateur du réseau de microélectrodes (1) peut être chargé sans fil par l'intermédiaire du dispositif de réception.

22. Réseau de microélectrodes selon l'une des revendications précédentes, **caractérisé en ce que** le réseau de microélectrodes (1) comporte un dispositif de transmission de signaux sans fil qui est conçu pour transmettre sans fil des signaux, notamment sous forme de données, du réseau de microélectrodes (1) vers un dispositif de réception de signaux et/ou pour transmettre sans fil des signaux, en particulier sous forme de données, d'un dispositif d'émission de signaux vers le réseau de microélectrodes (1).

23. Réseau de microélectrodes (1) selon l'une des revendications précédentes, **caractérisé en ce que** l'une, plusieurs ou la totalité des électrodes (2) sont respectivement intégrées dans une structure en colonnes (22) en relief, qui espace les électrodes (2) individuelles du plan du substrat du côté du capteur (16) .

24. Réseau de microélectrodes (1) selon la revendication 23, **caractérisé en ce que** l'une, plusieurs ou la totalité des structures en colonnes (19) sont constituées du matériau du substrat.

25. Réseau de microélectrodes (1) selon l'une des revendications précédentes, **caractérisé en ce que** l'une, plusieurs ou la totalité des structures en colonnes (19) présentent respectivement un rebord d'étanchéité circonférentiel (23) qui délimite latéralement la surface d'électrode active de l'électrode (2) intégrée à la structure en colonnes (22).

26. Réseau de microélectrodes (1) selon l'une des revendications précédentes, **caractérisé en ce que** des ouvertures traversantes (24) sont disposées dans le substrat (4), lesquelles conviennent pour permettre la diffusion de substances pharmacologiques dans le tissu et/ou pour introduire une ou plusieurs électrodes profondes pénétrantes (25) dans le tissu.

27. Réseau de microélectrodes selon la revendication 26, **caractérisé en ce que** plusieurs ou la totalité des ouvertures traversantes (24) sont disposées selon un motif fixe et prédéterminé de manière répartie sur l'étendue plane du substrat (4).

28. Réseau de microélectrodes (1) selon la revendication 26 ou 27, **caractérisé en ce qu'**une ou plusieurs électrodes de profondeur (25) ou au moins un réseau d'électrodes de profondeur (26) sont introduites dans les ouvertures traversantes (24) pour la mesure électrique simultanée de flux cérébraux de zones cérébrales profondes et/ou pour la stimulation électrique ainsi qu'optique de zones cérébrales profondes.

29. Dispositif comportant un réseau de microélectrodes selon l'une des revendications précédentes et au moins un dispositif de commande électronique, **caractérisé en ce que** le dispositif de commande électronique est couplé à l'un, plusieurs ou la totalité des composants électriques et/ou électroniques du réseau de microélectrodes (1).

30. Dispositif selon la revendication 29, **caractérisé en ce que** le dispositif de commande électronique est couplé par câble et/ou sans fil à l'un, plusieurs ou la totalité des composants électriques et/ou électroniques du réseau de microélectrodes (1).

31. Dispositif selon l'une des revendications 29 à 30, **caractérisé en ce que** le dispositif de commande électronique est au moins couplé à l'une, plusieurs ou la totalité des sources de lumière électriques (3) et est conçu pour commander le flux lumineux de l'une, de plusieurs ou de la totalité des sources de lumière électriques (3) couplées.

32. Dispositif selon la revendication 30, **caractérisé en ce que** le dispositif de commande électronique est conçu pour commander l'émission de lumière de l'une, de plusieurs ou de la totalité des sources de lumière électriques (3) couplées en fonction des composants de capteurs électriques et/ou électroniques du réseau de micro-électrodes (1), lesquelles sources respectivement un signal de capteur électrique en fonction d'au moins une grandeur physique influencée par la source de lumière électrique (3) ou les sources de lumière électriques (3), lequel signal est détecté par le composant de capteur respectif.
